# EUROPEAN PATENT SPECIFICATION

(11) **EP 3 171 894 B1**
(45) Date of publication and mention of the grant of the patent: **24.03.2021**
(21) Application number: 15749997.1
(22) Date of filing: 24.07.2015
(51) Int. Cl.: A61K 47/12, A61K 47/34, A61K 9/08, A61K 47/44, A61K 31/5377

(54) **NEUROKININ-1 RECEPTOR ANTAGONIST COMPOSITION FOR TREATMENT OF DISEASES AND CONDITIONS OF THE RESPIRATORY TRACT**
NEUROKININ-1-REZEPTORANTAGONISTENZUSAMMENSETZUNG ZUR BEHANDLUNG VON ERKRANKUNGEN UND ZUSTÄNDEN DER ATEMWEGE
COMPOSITION D'ANTAGONISTE DE RÉCEPTEUR DE NEUROKININE-1 POUR LE TRAITEMENT DE MALADIES ET D'AFFECTIONS DES VOIES RESPIRATOIRES

(30) Priority: 24.07.2014 US 201462028566 P
(43) Date of publication of application: 31.05.2017
(73) Proprietor: Plus Vitech, S.L., 41002 Sevilla (ES); Servicio Andaluz de Salud, 41071 Sevilla (ES)
(72) Inventor: LARA RUIZ, Antonio, E-41002 Sevilla (ES); SALINAS MARTÍN, Manuel Vicente, E-41002 Sevilla (ES)
(74) Representative: J A Kemp LLP
(86) International application number: PCT/EP2015/067006
(87) International publication number: WO 2016/012594

(56) References cited:
- EP-A1- 1 970 057
- WO-A1-2014/005606
- US-A1- 2013 317 016
- Anonymous: "NK1 receptor antagonist", Wikipedia, 23 September 2018 (2018-09-23), pages 1-10, XP055549834, Retrieved from the Internet: URL:https://en.wikipedia.org/wiki/NK1_rece ptor_antagonist [retrieved on 2019-01-31]
- GIOVANNI VITI ET AL: "Synthesis of a benzo[b]-1,5-naphthyridine derivative as a potential constrained NK1 receptor antagonist", TETRAHEDRON LETTERS, vol. 35, no. 32, 1 August 1994 (1994-08-01), pages 5939-5942, XP055461417, AMSTERDAM, NL ISSN: 0040-4039, DOI: 10.1016/S0040-4039(00)78224-4
- ATSUSHI YAMAMOTO ET AL: "A New Nonpeptide Tachykinin NK1 Receptor Antagonist Isolated from the Plants of Compositae", CHEMICAL AND PHARMACEUTICAL BULLETIN, vol. 50, no. 1, 1 January 2002 (2002-01-01), pages 47-52, XP055011736, DOI: 10.1248/cpb.50.47
- ACHARD A ET AL: "Perhydrothiopyranopyrroles derivatives : A novel series of potent and selective nonpeptide NK1 antagonists", BIOORGANIC & MEDICINAL CHEMISTRY LETTERS, PERGAMON, AMSTERDAM, NL, vol. 4, no. 5, 10 March 1994 (1994-03-10), pages 669-672, XP026637450, ISSN: 0960-894X, DOI: 10.1016/S0960-894X(01)80177-3 [retrieved on 1994-03-10]

## Description

### Field of the invention

The present invention relates to a composition comprising aprepitant, which is a neurokinin-1 (NK1) receptor antagonist, and at least one excipient which is olive oil. In addition, the present invention also relates to an administration form for administering a formulation comprising said composition. Furthermore, the present invention relates to a pharmaceutical composition for use in the treatment of at least one disease of the respiratory tract. Moreover, the invention also provides a method for producing said composition and said administration form, as well as a kit of parts comprising said composition and an administration device.

### Background to the invention

The present invention relates to a composition for administering aprepitant, which is a neurokinin-1 (NK1) receptor antagonist. An NK1 receptor antagonist is a type of receptor ligand that inhibits agonist-mediated (including inverse-agonist-mediated) responses upon binding to the NK1 receptor. The NK1 receptor is also known as the tachykinin receptor 1 (TACR1) or substance P receptor (SPR) and is a G protein-coupled receptor found in the central nervous system and peripheral nervous system.

NK1 receptor antagonists possess antidepressant, anxiolytic and antiemetic properties, and serve particular purpose in the prevention of nausea and vomiting associated with cancer chemotherapy.

Aprepitant (CAS 170729-80-3) is also known by the IUPAC name 5-([(2R,3S)-2-((R)-1-[3,5-*bis*(trifluoromethyl)phenyl]ethoxy)-3-(4-fluorophenyl)morpholino]methyl)-1H-1,2,4-triazol-3(2H)-one and may be represented by the following structural formula:

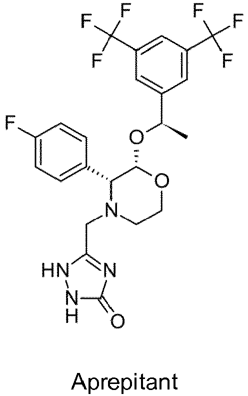

Aprepitant is an antiemetic compound, whereby said antiemetic effect is brought about by the fact that aprepitant blocks the NK1 receptor. Thus, aprepitant is used in a clinical setting for prevention of acute and delayed chemotherapy-induced nausea and vomiting (CINV) and for prevention of postoperative nausea and vomiting. Aprepitant may also be useful in the treatment of cyclic vomiting syndrome and late-stage chemotherapy-induced vomiting (CIV).

Aprepitant was approved by the FDA in 2003 and in many countries, is marketed as a formulation under the name Emend, wherein said Emend composition comprises, in addition to aprepitant, the following list of excipients:

| *Emend capsules* | *Excipient* |
|---|---|
| Capsule contents (comprising aprepitant) | Saccarose |
| | Microcrystalline cellulose (E460) |
| | Hydroxypropylcellulose (E463) |
| | Sodium lauryl sulfate |
| Capsule cover | Gelatine |
| | Titanium dioxide (E171) |
| | Ferric oxide yellow (E172) |

Alternatively, the following composition may be used:

| *Tablets* | *Excipient* |
|---|---|
| Tablet contents (comprising aprepitant) | Colloidal anhydrous silica |
| | Sodium lauryl sulfate |
| Tablet cover | Potassium hydroxide |
| | Ferric oxide black (E172) |
| | Lacquer |
| | Printing ink |

The aforementioned Emend composition is that which has been used in order to administer aprepitant orally. The capsule contents comprise individual molecules or particles of aprepitant which are covered with numerous molecules of cellulose which, upon ingestion, dissolve in the aqueous medium of the digestive tract and facilitate dissolution of aprepitant in said aqueous medium, as required for its absorption. In spite of this, the degree of absorption in the digestive tract (oral bioavailability) of the active ingredient is very low, at 67% in a capsule of 80 mg and at 59% in a capsule of 125 mg. In other words, the level of absorption is not only low but is further lowered by increasing the dose, such that higher doses do not increase the absorption in a linear manner, rather they decrease the level of absorption upon increasing the dose.

In this sense, Section 5.2 of the Summary of the Product Characteristics of Emend which is available in Spanish (published as Anexo I: Resumen de las Características del Producto) by Merck Sharp & Dohme states that:
*"aprepitant exhibits non-linear pharmacokinetics. Not only the clearance but also the absolute bioavailability diminishes upon increasing the dose".*

With respect to the absorption, the average máximum plasma concentration (Cₘₐₓ) of aprepitant is reached after approximately 4 hours (Tₘₐₓ). The oral administration of the capsule with a standard breakfast of approximately 800 Kcal causes an increase in up to 40% of the plasma concentration of aprepitant in the Area Under the Curve (AUC). However, according to the aforementioned Summary of the Product Characteristics of Emend, this increase is not considered of clinical interest.

The pharmacokinetics of aprepitant are non-linear in the interval of the clinical dose. In healthy young adults, the increase in the AUC_{0 - ∞} was 26% greater in proportion to the dose between the single doses of 80 mg and 125 mg administered in the postprandial state.

After the oral administration of a single dose of 125 mg of Emend on day 1 and 80 mg once a day on days 2 and 3, the AUC₀₋₂₄ₕ (average ± SD) was 19.6 ± 2.5 micrograms x h/mL and 21.2 ± 6.3 micrograms x h/mL on days 1 and 3, respectively. The Cₘₐₓ was 1.6 ± 0.36 micrograms/mL and 1.4 ± 0.22 micrograms/mL on days 1 and 3, respectively.

Thus, the oral administration of aprepitant not only results in poor absorption but is also inefficient since part of the absorbed active material is eliminated by the portal system upon reaching the liver from the digestive tract in the first hepatic stage when it is metabolised hepatically by cytochrome CYP3A4 (and possibly to a smaller degree with CYP1A2 and CYP2C19), as also stated in Section 5.2 *Metabolism* (*Metabolismo*) of the aforementioned Summary of the Product Characteristics, as follows:
*"Aprepitant is extensively metabolised. In healthy young adults, aprepitant represents approximately 19% of the plasma radioactivity over 72 hours after a single 100 mg intravenal dose of the propharmaco aprepitant-[C¹⁴], which indicates an important presence of metabolites in the plasma. In human plasma 12 metabolites of aprepitant have been identified. The metabolism of aprepitant is largely produced by oxidation of the morpholine ring and its side-chains, and its resulting metabolites were only weakly active. In vitro studies in which human hepatic microsomes were used indicated that aprepitant is principally metabolized by CYP3A4 and possibly with a lesser contribution through CYP1A2 and CYP2C19."*

Moreover, the oral administration of aprepitant varies with respect to the age, sex, presence in the patient of hepatic or renal insufficiency (wherein said variation may be clinically relevant or that conclusive evidence is not available), as disclosed in Section 5.2 *Pharmacokinetics in special populations* (*Farmacocinética en poblaciones especiales*) of the aforementioned Summary of the Product Characteristics, as follows:
*"Elderly: After oral administration of a single dose of 125 mg of Emend on day 1 and 80 mg once a day on days 2 and 5m the AUC₀₋₂₄ₕ of aprepitant was 21% greater on day 1 and* **36%** *greater on day 5 in elderly (≥ 65 years) in comparison with younger adults. The Cₘₐₓ was 10% greater on day 1 and 24% greater on day 5 in elderly in comparison with younger adults. These differences are not considered clinically significant. Emend does not require an altered dose in elderly patients.*
*Sex: After oral administration of a single dose of 125 mg of Emend, the Cₘₐₓ of aprepitant is 16% greater in women in comparison with men. The half-life of aprepitant is 25% greater in women in comparison with men and its Tₘₐₓ is produced in approximately the same time. These differences are not considered clinically significant. Emend does not require an altered dose as a function of sex.*
*Pediatric patients: The farmacokinetics of Emend have not been evaluated in patients of less than 18 years of age.*
*Hepatic insufficiency: Mild hepatic insufficiency (Child-Pugh score of 5 to* 6) *does not affect to a clinically relevant degree. It is not necessary to adjust the dose in patients with mild hepatic insufficiency. Conclusions related to the influence of moderate hepatic insufficiency (Child-Pugh score of 7 to 8) on the pharmacokinetics of aprepitant cannot be derived from the available data. Clinical or pharmacokinetic data on patients with severe hepatic insufficiency (Child-Pugh score >9) do not exist.*
*Renal insufficiency: A single dose of 240 mg of Emend was administered to patients with severe renal insufficiency (CrCI < 30 ml*/*min) and to patients with terminal nephropathy which required hemodialysis. In patients with severe renal insufficiency, the AUC*_{*0-*∞} *of total aprepitant (non-bonded and bonded to proteins) reduced by 21% and the Cₘₐₓ reduced by 32%, with respect to healthy patients. In patients with terminal nephropathy who had been subject to hemodialysis, the AUC_{0-∞} of total aprepitant reduced by 42% and the Cₘₐₓ reduced by 32%. Due to the modest decreases in the bonding of aprepitant to proteins in patients with renal disease, the AUC of the non-bonded pharmacologically-active drug was not observed to be significantly affected in those patients with renal insufficiency in comparison with healthy subjects. Haemodialysis carried out 4 or* 48 *hours after the administration did not have significant effects on the pharmacokinetics of aprepitant: less than 0.2% of the dose was recovered from the dialysate."*

Pharmaceutical compositions of aprepitant are disclosed in EP 1 970 057 A1, WO 2014/005606 A1 and US 2013/0317016 A1.

Thus, there is a need to provide a means for administering aprepitant which increases the bioavailability and efficiency of this drug, yet minimizes its metabolism, thereby allowing the administered dose to be more tightly controlled depending on the patient group.

### Brief description of the invention

The present invention relates to a pharmaceutical composition suitable for inhalation, wherein the pharmaceutical composition comprises a neurokinin-1 (NK1) receptor antagonist and at least one excipient, and the neurokinin-1 (NK1) receptor antagonist is aprepitant and the at least one excipient is olive oil.

In addition, the present invention relates to an administration form for administering a formulation, wherein said administration form is selected from: inhalant, nebulization, vapor, smoke or aerosol and comprises the composition of the present invention.

Moreover, the present invention relates to a composition for use as medicament or administration form for use as medicament, wherein said composition is defined according to the composition of the present invention and said administration form is defined according to the administration form of the present invention.

Furthermore, the present invention relates to a composition for use as medicament in the treatment of at least one disease of the respiratory tract, or administration form for use as medicament in the treatment of at least one disease of the respiratory tract, wherein said composition is defined according to the composition of the present invention and said administration form is defined according to the administration form of the present invention. In a preferred embodiment of the present invention, said at least one disease of the respiratory tract is selected from cancer, an inflammatory condition and an inflammatory-fibrosis condition.

Analogously, the present disclosure also relates to a method of treatment of a patient suffering from at least one disease of the respiratory tract which comprises the administration of an effective dose of the composition of the present invention. In a preferred embodiment said method of treatment comprises the administration of an effective dose of the composition of the present invention by using any of the administration forms of the present invention.

In addition, the present invention also relates to a kit of parts for administering a formulation, comprising:
i) a container comprising the composition according the present invention; and
ii) an administration device for administering said composition in any of the administration forms of the present invention from said container.

Moreover, the present invention relates to a method for producing the pharmaceutical composition according to the present invention, which comprises mixing:
a) a neurokinin-1 (NK1) receptor antagonist which is aprepitant; and
b) at least one excipient which is olive oil.

### Description of the invention

The aforementioned complex problem of the present invention is based on the recognition that the bioavailability and efficiency of aprepitant may be greatly increased by its administration as an inhalable composition comprising aprepitant and at least one excipient which is olive oil. It is assumed that the improved bioavailability and efficiency of said aprepitant composition derives from the hydrophobic nature of the aforementioned excipients which provide it with a high affinity for the lipid membranes of the respiratory tract (such as the surface-active lipoprotein (phospholipoprotein) complex of the pulmonary surfactant), thus allowing aprepitant to be brought into direct contact with said membranes for ready absorption. However, other mechanisms may also play a role in increasing the bioavailability and efficiency of aprepitant. Moreover, compositions comprising aprepitant and olive oil are shown to be effective for administration in inhalable administration forms.

In addition to aprepitant, other related neurokinin (NK1) receptor antagonists exist which may also benefit from an improved mode of administration. Said other NK1 receptor antagonists may be used but do not form part of the present invention. For example, an intravenous prodrug form of aprepitant, fosaprepitant (which has the tradenames Emend Injection and Ivemend) may be used in the present invention.

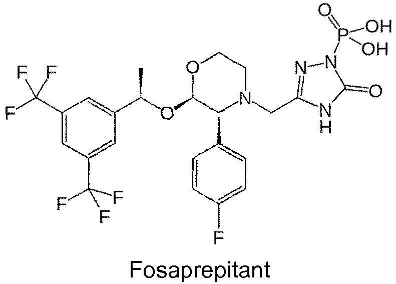

Similarly, casopitant (which has the trade names rezonic and zunrisa), maropitant (which has the trade name Cerenia), vofopitant (also known as vofopitant dihydrochloride), L-733,060, lanepitant (also known as LY-303870), vestipitant and L-732,138 are also NK1 receptor antagonists which may be used in the present invention.

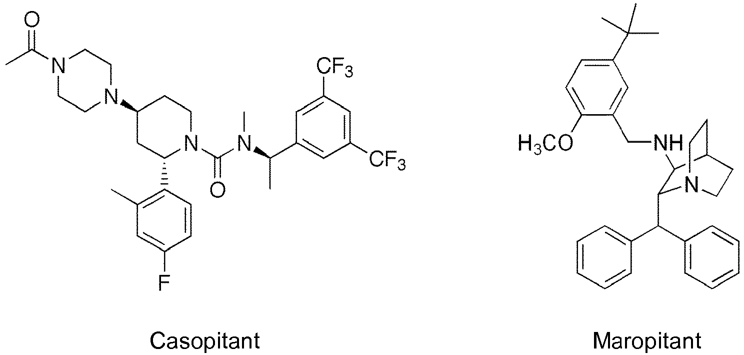

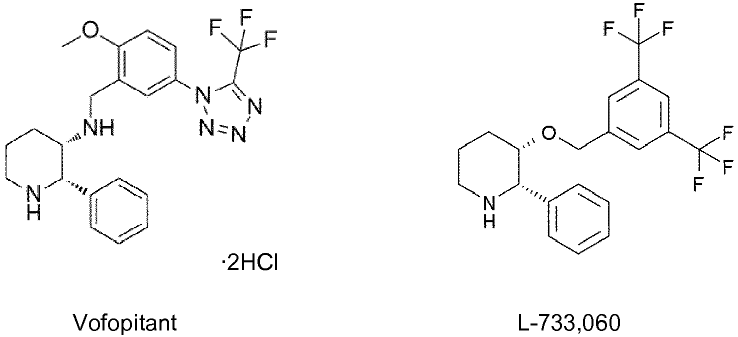

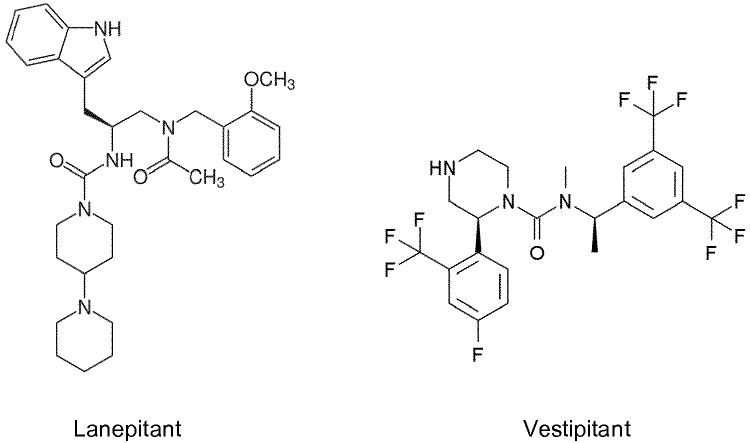

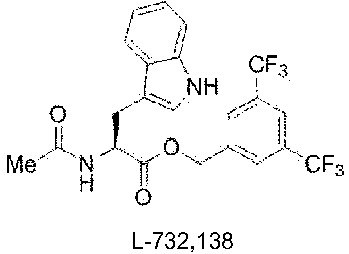

In the present invention, the concentration of NK1 receptor antagonist in the composition of the invention is between 0.001 mg/mL and 2000 mg/mL, preferably between 1 and 1000 mg/mL, more preferably between 5 and 250 mg/mL.

Olive oil is a fat obtained from plants of the family Oleaceae, in particular plants of the *Olea* genus, preferably *Olea europaea.* Preferably, olive oil is a fat obtained from the fruit of said plants, in particular from the olive. Said olive oil may be of extra virgin, virgin, lampante, refined or pomace olive oil quality or grades. As previously stated, one embodiment of the present invention relates to a composition comprising aprepitant and olive oil, preferably virgin olive oil, more preferably extra virgin olive oil.

Olive oil is composed of the mixed triglyceride esters of oleic acid (55 to 83%), palmitic acid (7.5 to 20%), linoleic acid (3.5 to 21%), stearic acid (0.5 to 5%) and α-linolenic acid (0 to 1.5%), mixed and/or non-mixed triglyceride esters of the aforementioned fatty acids.

In one embodiment of the composition, alimentary aromas and water, together with aprepitant (optionally with other active ingredients or excipients) make up the remainder of the composition.

The at least one aroma is a compound or composition that emits an odor which the human brain associates with a particular object or objects. Preferably the at least one aroma is a compound or composition that emits an odor which the human brain associates with tobacco or food, more preferably food, the odor thus being an alimentary odor. Furthermore preferably, the odor is an odor of fruit, apples, pears, strawberries, grapes, oranges, lemons, raspberries, cherries, bananas, peaches, plums, mango, pineapple, coffee, chocolate, cotton candy, cinnamon, vanilla, wintergreen, peppermint, hazelnuts and almonds.

Another aspect of the present invention relates to an administration form for administering a formulation comprising the composition of the present invention. The administration form may be any form that allows the composition of the present invention to be inhaled or forced along the airways of the respiratory tract as a gaseous dispersion of particles, wherein said particles are either in liquid (droplet) or solid (powder, especially micronized powder) form. In one embodiment, the administration form is selected from an inhalant, a nebulization, vapor, smoke or an aerosol. An aerosol may take the form of a spray and an inhalant may take the form of a pulverization. In a preferred embodiment, the administration form is selected from an inhalant, pulverization, spray or aerosol. In a more preferred embodiment, the administration form is selected from an inhalant, a nebulization or an aerosol, more preferably an inhalant or nebulization.

As such, another aspect of the present invention relates to a kit of parts, wherein said kit of parts comprises a container comprising the composition of the present invention, and an administration device for administering said composition in any of the forms of an inhalant, a nebulization, vapor, smoke or an aerosol. Said container may be in the form of a bottle, vial, ampoule, capsule, sachet, syringe or cartridge. Said administration device may be any device which can allow the composition of the present invention to form a fine dispersion in a gas or gas composition at ambient temperature and pressure. Said administration device may use said gas (such as oxygen or tetrafluoroethane) or said gas composition (such as air or an anaesthetic gas composition) at ambient temperature and pressure, or compressed and/or heated forms thereof, to form said administration form. Alternatively, the composition of the present invention or a formulation comprising it may be subjected to heating, combustion, ultrasonication or atomization so as to form a fine dispersion. In a preferred embodiment, the administration device is an inhaler, nebulizer, vaporizer, electronic cigarette, aerosol canister or bottle, atomizer or ventilator.

The administration form of the present invention comprises the composition of the present invention. In addition to comprising the NK1 receptor antagonist and excipient of the composition of the present invention, said administration form may additionally comprise at least one further excipient and/or carrier. Said further excipient and/or carrier may be an inert ingredient or ingredients. For example, the composition of the invention may be mixed with a carrier, diluted by a carrier, enclosed within a carrier, or adsorbed or absorbed on a granular solid carrier.

When the carrier serves as a diluent, it may be solid, semi-solid, or liquid material that acts as a vehicle, excipient, or medium for the active compound. Some examples of suitable carriers are the aforementioned vegetable oils, aforementioned mixed and/or non-mixed triglyceride esters and aforementioned fatty acids, but also include solvents such as alcohols (preferably ethanol), polyethylene glycols, vegetable glycerine, fatty acid monoglycerides (derived from the aforementioned fatty acids), mixed and/or non-mixed fatty acid diglycerides (derived from the aforementioned fatty acids), polyhydroxyethoxylated oils (preferably polyhydroxyethoxylated vegetable oils from the aforementioned vegetable oils, furthermore preferably polyhydroxyethoxylated olive oil), pentaerythritol fatty acid esters, water, and salt solutions such as isotonic saline. Other carriers such as lactose, terra alba, sucrose, cyclodextrin, amylose, magnesium stearate, talc, gelatin, agar, pectin, acacia, lower alkyl ethers of cellulose, silicic acid, fatty acid amines, polyoxyethylene, hydroxymethylcellulose, polyvinylpyrrolidone may also be used. The formulations may also include wetting agents, emulsifying and suspending agents, preserving agents, sweetening agents, flavouring agents or aromas (preferably alimentary aromas). In addition, the pharmaceutical compositions can be sterilized and mixed, if desired, with auxiliary agents, emulsifiers, salts for influencing osmotic pressure, buffers and/or colouring substances and the like, which do not deleteriously react with the NK1 receptor antagonist of the composition of the invention.

Thus, in one particularly preferred embodiment, the composition of the invention comprises aprepitant and olive oil suitable for administration as an inhaler spray, aerosol or nebulized administration form, using an inhaler or nebulizer as an administration device.

The administration form of the present invention allows the composition of the invention to reach all parts of the respiratory tract in human or animal patients, from the nasal passages to the larynx, pharynx, esophagus, trachae and lung, whereby the locations of the lung which may be reached include the bronchioles and alveoli. It is possible to treat all of the parts of the respiratory tract in the same manner using the composition of the invention.

As such, the composition of the present invention may be used to treat disease or conditions in human or animal patients. Optionally, said composition of the present invention is in one of the administration forms of the present invention. In one embodiment of the present invention, said disease or condition is at least one disease or condition of the respiratory tract. Preferably, said at least one disease or condition of the respiratory tract is a disease or condition which has an associated inflammatory component. In one more preferred embodiment of the present invention, said at least one disease of the respiratory tract is selected from cancer, an inflammatory condition and an inflammatory-fibrosis condition. In an even more preferred embodiment of the present invention, said at least one disease of the respiratory tract is a cancer selected from lung cancer, tracheal cancer, laryngeal cancer, pharyngeal cancer, cancer of the nasal cavities and esophageal cancer. Alternatively, in another even more preferred embodiment of the present invention said at least one disease or condition of the respiratory tract is an inflammatory condition selected from rhinitis, glossitis, buccal aphthous stomatitis, gingivitis, pharyngitis, laryngitis, bronchitis and alveolitis. In a still more preferred embodiment, said bronchitis is selected from allergic bronchitis, asthmatic bronchitis and infectious bronchitis, wherein said infectious bronchitis is preferably bacterial bronchitis or viral bronchitis. In another still more preferred embodiment, said alveolitis is selected from allergic alveolitis, asthmatic alveolitis and infectious alveolitis, wherein said infectious alveolitis is preferably bacterial alveolitis or viral alveolitis. On the other hand, in another even more preferred embodiment of the present invention said at least one disease or condition of the respiratory tract is an inflammatory-fibrosis condition selected from usual interstitial pneumonia, idiopathic interstitial pneumonias and pneumoconiosis.

In a preferred embodiment of the invention, said at least one disease or condition of the respiratory tract is selected from lung cancer, tracheal cancer, laryngeal cancer, pharyngeal cancer, cancer of the nasal cavities, esophageal cancer, rhinitis, glossitis, buccal aphthous stomatitis, gingivitis, pharyngitis, laryngitis, bronchitis, alveolitis, usual interstitial pneumonia, idiopathic interstitial pneumonias and pneumoconiosis.

In addition, the composition of the present invention may be used as an emetic in treating nausea and vomiting associated with cancer chemotherapy in human patients. In particular, the composition of the present invention may be used as an emetic in treating nausea and vomiting associated with cancer chemotherapy used in the treatment of the aforementioned cancers. Preferably, the cancer chemotherapy emesis is cisplatin-induced emesis.

Analogously, the present disclosure also relates to a method of treatment of a patient suffering from at least one disease of the respiratory tract which comprises the administration of an effective dose of the composition of the present invention. Optionally, said method of treatment is by using any of the administration forms of the present invention. In one preferred embodiment of the foregoing method of treatment, the at least one disease of the respiratory tract is selected from: cancer, an inflammatory condition and an inflammatory-fibrosis condition. In a more preferred embodiment of the method of treatment of the present invention, the at least one disease of the respiratory tract is cancer selected from lung cancer, tracheal cancer, laryngeal cancer, pharyngeal cancer, cancer of the nasal cavities and esophageal cancer. In another more preferred embodiment of the method of treatment of the present invention, the at least one disease of the respiratory tract is an inflammatory condition selected from rhinitis, glossitis, buccal aphthous stomatitis, gingivitis, pharyngitis, laryngitis, bronchitis and alveolitis. In yet another more preferred embodiment of the method of treatment of the present invention, the at least one disease of the respiratory tract is an inflammatory-fibrosis condition selected from usual interstitial pneumonia, idiopathic interstitial pneumonias and pneumoconiosis.

In the present invention, the effective dose of the composition of the present invention is an inhaled dose of between 0.01 and 1.0 mg/kg of bodyweight per day, preferably between 0.05 and 0.5 0 mg/kg of bodyweight per day, more preferably between 0.05 and 0.4 0 mg/kg of bodyweight per day, still more preferably between 0.1 and 0.20 mg/kg of bodyweight per day. Said dose may be distributed into between 1 and 5 sub-doses inhaled at intervals throughout the day, preferably between 2 and 4 subdoses, more preferably 3 subdoses.

The present invention also relates to a method for producing the pharmaceutical composition of the present invention, which comprises mixing an NK1 receptor antagonist according to the foregoing, and olive oil. Optionally said method additionally comprises a step c) of mixing at least one further excipient and/or carrier with said NK1 receptor antagonist and said olive oil. Said method may involve a step of heating, agitation, centrifugation and/or filtration in order to ensure homogeneity of the resulting mixture.

The present invention further relates to a method for producing the administration form according to the present invention, which comprises the foregoing method for producing the pharmaceutical composition according to the present invention and subsequently finely-dividing, nebulizing, vaporizing, atomizing and/or aerosolizing said pharmaceutical composition.

### Experimental results

In the following Examples, aprepitant was used either as a pure compound, as were the other NK1 receptor antagonists, L-732,138, vestipitant and casopitant, or as a component of a commercial formulation (Emend) microencapsulated in cellulose. Examples not referring to aprepitant and olive oil are reference examples.

### Example 1

i) Pure aprepitant (10 mg) was measured into a vial, to which was added extra virgin olive oil (1 mL). This procedure was repeated in separate vials, each time increasing the amount of aprepitant so as to obtain compositions with a potential range of different concentrations of aprepitant of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL and 125 mg/mL. The resulting mixtures were dispersed by gentle agitation at ambient temperature (25 °C) over a time of from 1 - 24 hours, and allowed to settle after which time clear solutions were obtained.
ii) Aprepitant microencapsulated in cellulose was measured into a vial such that said vial comprised 10 mg of aprepitant, to which was added extra-virgin olive oil (1 mL). This procedure was repeated in separate vials, each time increasing the amount of aprepitant microencapsulated in cellulose so as to obtain compositions with a potential range of different concentrations of aprepitant of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL and 125 mg/mL. The resulting mixtures were dispersed by gentle agitation at ambient temperature (25 °C) over a time of from 1 - 24 hours. The resulting suspensions comprising white solids were centrifuged in order to obtain a clear solution.
Aprepitant was found to be completely solubilized in the compositions resulting from i) and ii). Said compositions were each placed (together with approximately 5 mL of air) in syringe barrels. Upon applying pressure to the piston (plunger) of each syringe each of said compositions formed a fine aerosol upon exiting the nozzles thereof.
iii) In order to prove that NK1 receptor antagonists other than aprepitant dissolve in olive oil, the saturation concentration of various NK1 receptor antagonists as solutes in olive oil (solvent) was determined. To this end, increasing quantities of various NK1 receptor antagonists, including aprepitant, as active ingredients were separately mixed in a 10 mL volume of olive oil in order to formulate compositions with a potential range of different concentrations of NK1 receptor antagonists of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL and 125 mg/mL. Each of said mixtures was continuously and gently stirred for 10 minutes at ambient temperature (25 °C) and then maintained at rest for 60 minutes. The saturation concentration was considered to be that which was immediately lower in the aforementioned series than a concentration where deposition was observed, but in which no deposition of the active ingredient was observed. The data of the saturation concentration are shown in Table 1.

**Table 1. Saturation concentrations for different solutes in olive oil**

| **Solute** | **Molecular mass (g/mol)** | **Saturation concentration (nM)** |
|---|---|---|
| Aprepitant | 534.4 | 60 |
| L-732,138 | 434.4 | 80 |
| Vestipitant | 545.4 | 60 |
| Casopitant | 616.3 | 40 |

iv) In order to prove that the capacity of aprepitant and other NK1 receptor antagonists to dissolve is improved with the addition of an alcohol, the saturation concentration of various NK1 receptor antagonists as solutes in a mixed solvent comprising olive oil was determined. To this end, increasing quantities of various NK1 receptor antagonists, including aprepitant, as active ingredients were separately mixed in a solvent mixture consisting of a 9 mL volume of olive oil and 1 mL of ethanol in order to formulate compositions with a potential range of different concentrations of NK1 receptor antagonists of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL and 125 mg/mL. Each of said mixtures was continuously and gently stirred for 10 minutes at ambient temperature (25 °C) and then maintained at rest for 60 minutes. The saturation concentration was considered to be that which was immediately less than a concentration where deposition was observed, but in which no deposition of the active ingredient was observed. The data of the saturation concentrations of different solutes in olive oil:ethanol (9:1) are shown in Table 2.

**Table 2. Saturation concentrations for different solutes in olive oil:ethanol (9:1)**

| **Solute** | **Molecular mass (g/mol)** | **Saturation concentration (nM)** |
|---|---|---|
| Aprepitant | 534.4 | 80 |
| L-732,138 | 434.4 | 100 |
| Vestipitant | 545.4 | 80 |
| Casopitant | 616.3 | 65 |

v) In order to prove that oils of types other than olive oil can act as solvents of aprepitant and other NK1 receptor antagonists, the saturation concentration of various NK1 receptor antagonists as solutes in various oils was determined. To this end, increasing quantities of various NK1 receptor antagonists, including aprepitant, as active ingredients were separately mixed in a 10 mL volume of various oils in order to formulate compositions with a potential range of different concentrations of NK1 receptor antagonists of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL and 125 mg/mL. Each of said mixtures was continuously and gently stirred for 10 minutes at ambient temperature (25 °C) and then maintained at rest for 60 minutes. The saturation concentration was considered to be that which was immediately less than a concentration where deposition was observed, but in which no deposition of the active ingredient was observed. The data of the maximum concentration of dissolution are shown in Table 3.

**Table 3. Saturation concentrations for different solutes in different oils**

| **Solute** | **Molecular mass (g/mol)** | **Saturation concentration (nM)** | | |
|---|---|---|---|---|
| | | **Soy oil** | **Sunflower oil** | **Rapeseed oil** |
| Aprepitant | 534.4 | 60 | 70 | 80 |
| L-732,138 | 434.4 | 90 | 80 | 90 |
| Vestipitant | 545.4 | 80 | 80 | 70 |
| Casopitant | 616.3 | 60 | 60 | 70 |

### Example 2

The following two compositions were formulated such that the type and amount of excipients comprised therein are analogous to those of the "inhaler spray" denominated "Budesonide Aldo-union".
i) Pure aprepitant (10 mg) was measured into a vial, to which was added a solution of oleic acid (0.69 mL) in ethanol (0.31 mL), *i.e.* a 69% (volume/volume) ethanolic solution of oleic acid. This procedure was repeated in separate vials, each time increasing the amount of aprepitant so as to obtain compositions with a potential range of different concentrations of aprepitant of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL and 125 mg/mL. The resulting mixtures were dispersed by gentle agitation at ambient temperature (25 °C) over a time of from 1 - 24 hours, after which time clear solutions were obtained.
ii) Aprepitant microencapsulated in cellulose was measured into a vial such that said vial comprised 10 mg of aprepitant, to which was added a solution of oleic acid (0.69 mL) in ethanol (0.31 mL), *i.e.* a 69% ethanolic solution of oleic acid. This procedure was repeated in separate vials, each time increasing the amount of aprepitant microencapsulated in cellulose so as to obtain compositions with a potential range of different concentrations of aprepitant of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL and 125 mg/mL. The resulting mixtures were dispersed by gentle agitation at ambient temperature (25 °C) over a time of from 1 - 24 hours. The resulting suspensions comprising white solids were centrifuged in order to obtain a clear solution.

Aprepitant was found to be completely solubilized in the compositions resulting from i) and ii). Said compositions were each placed in a pressurized, metered-dose inhaler and nebulized into a fine aerosol.

### Example 3

i) Pure aprepitant (10 mg) was measured into a vial, to which was added a solution of polyethyleneglycol (0.75 mL), vegetal glycerine (0.15 mL) and apple aroma (0.01 mL) in water (0.09 mL). This procedure was repeated in separate vials, each time increasing the amount of aprepitant so as to obtain compositions with a potential range of different concentrations of aprepitant of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL and 125 mg/mL. The resulting mixtures were dispersed by gentle agitation at ambient temperature (25 °C) over a time of from 1 - 24 hours, after which time clear solutions were obtained.
ii) Aprepitant microencapsulated in cellulose was measured into a vial such that said vial comprised 10 mg of aprepitant, to which was added a solution of polyethyleneglycol (0.75 mL), vegetal glycerine (0.15 mL) and apple aromas (0.01 mL) in water (0.09 mL). This procedure was repeated in separate vials, each time increasing the amount of aprepitant microencapsulated in cellulose so as to obtain compositions with a potential range of different concentrations of aprepitant of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL and 125 mg/mL. The resulting mixtures were dispersed by gentle agitation at ambient temperature (25 °C) over a time of from 1 - 24 hours. The resulting suspensions comprising white solids were centrifuged in order to obtain a clear solution.

Aprepitant was found to be completely solubilized in the compositions resulting from i) and ii). Said compositions were each placed in an electronic cigarette and vaporised.

### Example 4

This example demonstrates that the administration of NK1 receptor antagonists is more efficient in the lungs when administered by inhalation of an oil-alcohol mixture in which it is dissolved, than when administered orally. Herein, New Zealand albino rabbits (New Zealand White Rabbits) with an initial average weight of approximately 3 kg were used. The rabbits were fed *ad libitum* and were monitored daily to check that signs of toxicity had not appeared.

To assess its efficiency on the lungs when administered orally, aprepitant was administered to a group of 3 of said rabbits (group 1) in an oral dose of 10 mg/kg body weight per day, to another group of 3 rabbits (group 2) in an oral dose of 40 mg/kg body weight per day, to a third group (group 3) in an oral dose of 80 mg/kg body weight per day and to a fourth group (group 4) in an oral dose of 100 mg/kg body weight per day.

In parallel, a mixture consisting of aprepitant, as the active ingredient, and olive oil, as a solvent, was prepared. This mixture was administered as an aerosol to the airways of a group of 3 of said rabbits (Group 5) in a dose of 0.05 mg/kg body weight per day, to another group of three rabbits (Group 6) in a dose of 0.1 mg/kg body weight per day, to another group of three rabbits (group 7) in a dose of 0.2 mg/kg body weight per day and to another group of three rabbits (Group 8) in a dose of 0.4 mg/kg body weight per day. The mixture was administered as an aerosol by means of a pediatric inhalation mask connected to a nebulising system with an air compressor, wherein said aerosol and nebulising system had the following characteristics: particle size of 0.5 to 10 micrometres, average particle size 4 micrometres, compressor pressure range 30 to 36 psi (210 to 250 KPa/2.1 to 2.5 bar), operating pressure range 8 to 16 psi (50 to 100 kPa/0.5 to 1.0 bar), flow rate 6 to 8 litres/min.

After seven days of treatment all animals were euthanized and necropsied. Five lung tissue samples were taken from each animal, each corresponding to tissue from the five lung lobes (right superior, right middle, right inferior, left superior and left inferior). Samples were taken at the peripheral level of the lung parenchyma, just below the pleural surface. Tissue samples were also taken from the nasal cavity, pharynx, larynx and bronchi. In all samples the concentration of aprepitant was determined by HPLC.

The concentration of aprepitant measured in lung samples from the rabbits administered orally with aprepitant at doses of 10, 40, 80 and 100 mg/kg per day, is respectively shown in Tables 4 to 7 for Groups 1 to 4. On the other hand, the concentration of aprepitant measured in lung samples from rabbits administered with aprepitant by inhalation at doses of 0.05, 0.1, 0.2 and 0.4 mg/kg per day, is respectively shown in Tables 8 to 11 for Groups 5 to 6. LSPD, LMDP, LIPD, LSPI and LIPI respectively correspond to the concentration of aprepitant measured in the right superior lobe, right middle lobe, right inferior lobe, left superior lobe and left inferior lobe of the lungs in each of the rabbits (cases). The concentrations are expressed in micrograms of aprepitant per gram of lung tissue. The average is the arithmetic average calculated for each row or column of each table. The SD is the standard deviation, calculated for each row or column of each table.

**Table 4. Lung concentration of orally-administered aprepitant in Group 1.**

| | **LSPD** | **LMPD** | **LIPD** | **LSPI** | **LIPI** | **Average ± SD** |
|---|---|---|---|---|---|---|
| **Case 1** | 0.4 | 1.4 | 0.9 | 1.0 | 1.4 | 1.02 ± 0.41 |
| **Case 2** | 1.2 | 1.5 | 1.0 | 0.5 | 1.1 | 1.06 ± 0.36 |
| **Case 3** | 1.1 | 1.1 | 0.6 | 0.5 | 1.5 | 0.96 ± 0.41 |
| **Average ± SD** | 0.9 ± 0.4 | 1.3 ± 0.2 | 0.8 ± 0.2 | 0.6 ± 0.2 | 1.3 ± 0.2 | |

**Table 5. Lung concentration of orally-administered aprepitant in Group 2.**

| | **LSPD** | **LMPD** | **LIPD** | **LSPI** | **LIPI** | **Average ± SD** |
|---|---|---|---|---|---|---|
| **Case 1** | 4.1 | 2.9 | 3.1 | 4.2 | 2.8 | 3.4 ± 0.68 |
| **Case 2** | 3.3 | 5.1 | 4.2 | 3.6 | 4.0 | 4.04 ± 0.69 |
| **Case 3** | 3.2 | 4.2 | 3.3 | 5.3 | 5.4 | 4.2 ± 1.05 |
| **Average ± SD** | 3.5 ± 0.4 | 4.0 ± 1.1 | 3.5 ± 0.5 | 4.3 ± 0.8 | 4.0 ± 1.3 | |

**Table 6. Lung concentration of orallv-administered aprepitant in Group 3.**

| | **LSPD** | **LMPD** | **LIPD** | **LSPI** | **LIPI** | **Average ± SD** |
|---|---|---|---|---|---|---|
| **Case 1** | 4.2 | 5.5 | 6,3 | 5.1 | 3.4 | 4.9 ± 1.13 |
| **Case 2** | 3.3 | 5.3 | 5,3 | 5.1 | 4.3 | 4.6 ± 0.86 |
| **Case 3** | 4.9 | 4.8 | 5,1 | 3.9 | 3.6 | 4.4 ± 0.67 |
| **Average ± SD** | 4.1 ± 0.8 | 5.2 ± 0.3 | 5.5 ± 0.6 | 4.7 ± 0.6 | 3.7 ± 0.4 | |

**Table 7. Lung concentration of orally-administered aprepitant in Group 4.**

| | **LSPD** | **LMPD** | **LIPD** | **LSPI** | **LIPI** | **Average ± SD** |
|---|---|---|---|---|---|---|
| **Case 1** | 4.8 | 5.1 | 5.9 | 5.2 | 4.8 | 5.16 ± 0.45 |
| **Case 2** | 4.3 | 5.4 | 6.1 | 4.5 | 5.1 | 5.08 ± 0.72 |
| **Case 3** | 5.1 | 5.8 | 4.9 | 4.3 | 6.1 | 5.24 ± 0.72 |
| **Average ± SD** | 4.7 ± 0.4 | 5.4 ± 0.3 | 5.6 ± 0.6 | 4.6 ± 0.4 | 5.3 ± 0.6 | |

**Table 8. Lung concentration of aprepitant administered by inhalation in Group 5.**

| | **LSPD** | **LMPD** | **LIPD** | **LSPI** | **LIPI** | **Average ± SD** |
|---|---|---|---|---|---|---|
| **Case 1** | 1.4 | 1.6 | 1.5 | 1.5 | 1.5 | 1.5 ± 0.07 |
| **Case 2** | 1.5 | 1.5 | 1.6 | 1.5 | 1.4 | 1.5 ± 0.07 |
| **Case 3** | 1.4 | 1.5 | 1.5 | 1.4 | 1.5 | 1.4 ± 0.05 |
| **Average ± SD** | 1.4 ± 0.05 | 1.5 ± 0.05 | 1.5 ± 0.05 | 1.4 ± 0.05 | 1.4 ± 0.05 | |

**Table 9. Lung concentration of aprepitant administered by inhalation in Group 6.**

| | **LSPD** | **LMPD** | **LIPD** | **LSPI** | **LIPI** | **Average ± SD** |
|---|---|---|---|---|---|---|
| **Case 1** | 2.8 | 2.9 | 2.9 | 2.7 | 2.8 | 2.8 ± 0.08 |
| **Case 2** | 2.9 | 2.9 | 2.8 | 2.8 | 2.9 | 2.8 ± 0.05 |
| **Case 3** | 2.9 | 2.9 | 2.8 | 2.8 | 2.8 | 2.8 ± 0.05 |
| **Average ± SD** | 2.8 ± 0.05 | 2.9 ± 0 | 2.8 ± 0.05 | 2.7 ± 0.05 | 2.8 ± 0.05 | |

**Table 10. Lun concentration of aprepitant administered by inhalation in Group 7.**

| | **LSPD** | **LMPD** | **LIPD** | **LSPI** | **LIPI** | **Average ± SD** |
|---|---|---|---|---|---|---|
| **Case 1** | 5.9 | 6.0 | 6.0 | 6.1 | 6.0 | 6.0.± 0.07 |
| **Case 2** | 6.0 | 6.1 | 6.0 | 5.9 | 5.9 | 5.9 ± 0.08 |
| **Case 3** | 6.1 | 6.0 | 6.0 | 6.1 | 5.9 | 6.02 ± 0.08 |
| **Average ± SD** | 6.0 ± 0.1 | 6.03 ± 0.05 | 6.0 ± 0 | 6.03 ± 0.1 | 5.9 ± 0.05 | |

**Table 11. Lung concentration of aprepitant administered by inhalation in Group 8.**

| | **LSPD** | **LMPD** | **LIPD** | **LSPI** | **LIPI** | **Average ± SD** |
|---|---|---|---|---|---|---|
| **Case 1** | 10.0 | 10.1 | 10.4 | 10.2 | 10.1 | 10.1 ± 0.15 |
| **Case 2** | 9.8 | 9.9 | 10.0 | 10.0 | 9.9 | 9.9 ± 0.08 |
| **Case 3** | 10.1 | 10.0 | 9.9 | 9.9 | 10.0 | 9.9 ± 0.08 |
| **Average ± SD** | 9.9 ± 0.1 | 10.0 ± 0.1 | 10.1 ± 0.2 | 10.0 ± 0.1 | 10.0 ± 0.1 | |

In conclusion, administration of aprepitant by inhalation is more efficient than oral administration because similar concentrations of this drug may be obtained in the lung at much lower doses when administered by inhalation as opposed to oral administration. In addition, the distribution of aprepitant in the lung is more homogeneous when administered by inhalation rather than orally, as less variability in the measured concentrations is observed between the cases studied and, in each specific case, between different lung lobes. Furthermore, the concentration of aprepitant measured in the lungs is proportional to the dose administered by inhalation, whereas when aprepitant is administered orally the relationship between dose and concentration in lung tissue is non-linear and not proportional. In samples collected from the nasal cavity, larynx, pharynx and bronchi, similar results were obtained, such that administration of aprepitant by inhalation is more efficient (it achieves a concentration of the drug at lower dose), more homogeneous (with less variability between studied subjects) and proportional to the administered dose.

### Example 5

This example demonstrates that aprepitant and other NK1 receptor antagonists which are formulated in an oil-alcoholic solution exhibit greater efficacy in the treatment of respiratory tract diseases such as cancer (e.g. lung, tracheal, laryngeal, pharyngeal, esophageal, nasal cavity cancers, *etc*.), inflammatory diseases of the respiratory tract (*e.g.* rhinitis, pharyngitis, laryngitis, bronchitis, *etc*.), and inflammatory-fibrosis conditions (*e.g.* interstitial pneumonia, pneumoconiosis, *etc*.). It is known that the factors TGF-α, TGF-β1, TGF-β2, TGF-β3, SPARC, MMP-3; MMP-7, MMP-9, MMP-11, MMP-13 and MMP-14, TGF-β, NF-kB, EGF, MMP-9, VEGF and TNF-α which are produced by lung cells (pneumocytes, fibroblasts and vascular endothelial cells) and inflammatory cells (macrophages, lymphocytes and polymorphonuclear leukocytes) are involved in the pathophysiology and development of said respiratory tract diseases. In the present example the administration of aprepitant by inhalation is demonstrated to reduce the production of these factors, thus rendering it useful in the treatment of these diseases. In addition, it is demonstrated that administration of aprepitant by inhalation reduces the histological signs of disease. Herein, 27 New Zealand albino rabbits (New Zealand White Rabbit) with an initial average weight about 3 kg were used in this example. All subjects were administered an aerosol of a solution consisting of a mixture of water and sodium hypochlorite (35 g sodium hypochlorite per liter of water) at a rate of 5 mL of said solution by inhalation three times a day for 7 days (days 1 to 7 of experimentation). Subsequently, from days 3 to 7, a group of 3 rabbits (Group 1) was administered an oral dose of 10 mg/kg body weight per day, another group of 3 rabbits (Group 2) was administered an oral dose of 40 mg/kg body weight per day, a third group (Group 3) was administered an oral dose of 80 mg/kg body weight per day and a fourth group (Group 4) was administered an oral dose of 100mg/kg body weight per day.

In parallel, a mixture consisting of aprepitant as the active ingredient and olive oil as a solvent was prepared. This mixture was administered as an aerosol by inhalation, also from days 3 to 7, to a group of 3 rabbits (group 5) at a dose of 0.05 mg/kg body weight per day, to another group of three rabbits (Group 6) at a dose of 0.1 mg/kg body weight per day, to another group of three rabbits (Group 7) at a dose of 0.2 mg/kg body weight per day and to another group of three rabbits (Group 8) at a dose of 0.4 mg/kg body weight per day. A placebo consisting only of olive oil was administered from days 3 to 7 to a control group of 3 rabbits (placebo group). In parallel, 3 rabbits were kept under identical conditions but were not administered any treatment nor the aforementioned sodium hypochlorite solution (control group). The mixture was administered as an aerosol by means of a pediatric inhalation mask connected to a nebulising system with an air compressor, wherein said aerosol and nebulising system had the following characteristics: particle size of 0.5 to 10 micrometres, average particle size 4 micrometres, compressor pressure range 30 to 36 psi (210 to 250 KPa/2.1 to 2.5 bar), operating pressure range 8 to 16 psi (50 to 100 kPa/0.5 to 1.0 bar), flow rate 6 to 8 litres/min.

On day 7 of the experiment all animals were euthanized and necropsied. Five lung tissue samples were taken from each animal, each corresponding to tissue from the five lung lobes (right superior, right middle, right inferior, left superior and left inferior). Samples were taken at the peripheral level of the lung parenchyma, just below the pleural surface. Tissue samples were also taken from the nasal cavity, pharynx, larynx and bronchi. Immunohistochemistry studies were performed for all samples to evaluate the levels of the markers TGF-α, TGF-β1, TGF-β2, TGF-β3 SPARC, MMP-3; MMP-7, MMP-9, MMP-11, MMP-14, TGF-β, TNF-α and VEGF.

In said immunohistochemistry studies, a sample of each sample was dehydrated by treatment with increasing concentrations of ethanol and finally xylene. Subsequently said dried samples were embedded in paraffin, thus creating a block. Said paraffin blocks were cut on a microtome to a thickness of 5 µm, and the resulting sections (slices) were placed on slides suitable for conducting immunohistochemistry techniques. Subsequently, the sections were deparaffinised by immersion in xylene and then rehydrated through immersion in a series of solutions containing decreasing concentrations of ethanol and, finally, water. Subsequently, these samples were subjected to 10 times atmospheric pressure (10.1 bar) in citrate buffer at pH 6.0, in order to obtain greater exposure to antigens.

The samples were then allowed to cool to room temperature over 10 minutes. Endogenous peroxidase activity was blocked by treatment with 3% hydrogen peroxide over 30 min at room temperature. After washing the samples with 0.05 M Tris buffer, they were incubated with 10 % non-immune pig serum over 30 minutes at room temperature. In order to verify the expression of NK1 receptors, the cell samples were incubated in the presence of anti-NK1 antibodies (S8305, Sigma-Aldrich) diluted 1:1000 at 4 °C overnight. After this time they were washed in 0.05M Tris buffer at room temperature. Subsequently, Envision System-HRP (Dako) reagents were added for 30 min at room temperature. Once this time had expired, the samples were again washed with 0.05 M Tris buffer, and immunoreactivity was visualized by light microscopy with a chromogenic solution with 3,3'-diaminobenzidine (DAB+; Dako, USA). In order to differentiate the cell nuclei, these were lightly stained with haematoxylin. Samples that were not incubated with the primary antibody, but wherein this was replaced by a non-immune serum were used as negative controls. In order to evaluate markers in these immunohistochemical assays, specific primary antibodies were used against them at a concentration 1:1000, as detailed in Table 12. Furthermore, for each of the sampled sections were placed on slides and stained with haematoxylin-eosin before subsequently being deparaffinised by immersion in xylene and then rehydrated through immersion in a series of solutions containing decreasing concentrations of ethanol and, finally, immersing them in water, eosin (20 seconds) and hematoxylin (40 seconds). All experiments were performed in sextuplicate. In order to evaluate the degree of immunostaining in each of the six sections, a cell-count was performed in 20 high-power fields (400x) using an Olympus brand microscope (CX31 model). The total number of cells and the number of cells displaying immunostaining were counted in each one of the fields in order to subsequently determine the percentage of cells displaying said immunostaining. In the sections (slices) stained with hematoxylin/eosin the area occupied by fibrosis was assessed.

**Table 12. Antibodies used in immunohistochemical assays.**

| **Antibody** | **Reference code** | **Supplier** | **Characteristics** |
|---|---|---|---|
| TGFα | SAB4502953 | Sigma-Aldrich | Rabbit polyclonal |
| TGFβ1 | SAB4502954 | Sigma-Aldrich | Rabbit polyclonal |
| TGFβ2 | SAB4502956 | Sigma-Aldrich | Rabbit polyclonal |
| TGFβ3 | SAB4502957 | Sigma-Aldrich | Rabbit polyclonal |
| MMP-7 | SAB4501894 | Sigma-Aldrich | Rabbit polyclonal |
| MMP-9 | SAB4501896 | Sigma-Aldrich | Rabbit polyclonal |
| MMP-11 | SAB4501898 | Sigma-Aldrich | Rabbit polyclonal |
| MMP-14 | SAB4501901 | Sigma-Aldrich | Rabbit polyclonal |
| TNF-α | ab199013 | Abcam | Mouse monoclonal |
| VEGF | ab1316 | Abcam | Mouse monoclonal |

The results obtained show the presence of all the markers which were analysed in the samples derived from the control group and that the NK1 receptor agonist, aprepitant, induces a partial reduction thereof when administered orally. In this regard, the use of non-peptide NK1 receptor antagonists prevent the development and progression of said respiratory tract diseases.

In particular, the results shown in Table 13 show an increase of all the markers analysed in the samples derived from the placebo group, when compared to the control group, and that oral treatment with aprepitant reduces the presence of these markers. However, in Table 14 it can be seen that for groups treated with the NK1 receptor agonist, aprepitant by inhalation, this reduction in the levels of markers is significantly greater, if not complete, despite much lower doses having been administered. In this regard, the use of non-peptide NK1 receptor antagonists by inhalation prevents the development and progression of said respiratory tract diseases. In addition, this reduction is more homogeneous than when administered orally and is more predictable because it is proportional to the dose administered. Furthermore the use of NK1 receptor antagonists by inhalation is more efficient than the oral use of these antagonists, since much lower doses are necessary to achieve the same results.

**Table 13. Percent increase in marker for the placebo group and Groups 1 to 4 with respect to the control group ± SD in lung tissue.**

| | **%Med P/C** | **%Med G1/C** | **%Med G2/C** | **%Med G3/C** | **%Med G4/C** |
|---|---|---|---|---|---|
| **TGFα** | 2,285.12 68.71 ± | 1,717.76 123.54 ± | 1,308.73 ± 123.54 | 1,257.29 14.95 ± | 1,195.15 69.13 ± |
| **TGFβ1** | 1,636.51 16.26 ± | 1,277.48 73.42 ± | 990.28 ± 73.42 | 858.27 19.26 ± | 759.25 30.90 ± |
| **TGFβ2** | 1,025.49 ±45.24 | 775.23 57.52 ± | 582.05 ± 57.52 | 475.25 9.62 ± | 438.55 ±32.19 |
| **TGFβ3** | 1,067.12 6.04 ± | 835.84 54.48 ± | 655.71 ± 54.48 | 496.55 28.31 ± | 374.47 10.90 ± |
| **MMP-7** | 455.98 28.24 ± | 345.90 33.38 ± | 325.50 ± 30 | 220.38 8.80 ± | 184.56 4.35 ± |
| **MMP-9** | 733.62 103.31 ± | 566.19 ±24.64 | 384.44 ± 24.64 | 338.57 59.10 ± | 326.02 17.14 ± |
| **MMP-11** | 633.33 48.27 ± | 522.19 35.99 ± | 416.18 ± 35.99 | 339.86 54.57 ± | 251.62 39.25 ± |
| **MMP-14** | 865.63 43.40 ± | 683.47 16.76 ± | 569.37 ± 16.76 | 441.85 25.65 ± | 354.84 51.52 ± |
| **TNF-α** | 1,355.68 183.07 ± | 859.73 92.78 ± | 685.01 ± 92.78 | 607.15 65.62 ± | 463.11 103.08 ± |
| **VEGF** | 921,47 72,63 ± | 655.77 56.07 ± | 512.28 ± 56.07 | 458.30 45.95 ± | 342.93 15.74 ± |
| **Fibrosis** | 1,328.57 265.80 ± | 1,272.22 76.38 ± | 683.33 ± 76.38 | 611.11 153.96 ± | 516.67 125.83 ± |

| | | | | | |
|---|---|---|---|---|---|
| %Med P/C: percent increase in marker for the group treated with placebo with respect to the control group ± SD; %Med G1/C: percent increase in marker for the Group 1 with respect to the control group ± SD; %Med G2/C: percent increase in marker for the Group 2 with respect to the control group ± SD; %Med G3/C: percent increase in marker for the Group 3 with respect to the control group ± SD; %Med G4/C: percent increase in marker for the Group 4 with respect to the control group ± SD. | | | | | |

**Table 14. Percent increase in marker for the placebo group and Groups 5 to 8 with respect to the control group ± SD in lung tissue.**

| | **%Med P/C** | **%Med G5/C** | **%Med G6/C** | **%Med G7/C** | **%Med G8/C** |
|---|---|---|---|---|---|
| **TGFα** | 2,285.12 ± 68.71 | 1,972,41 ± 32,92 | 937.00 ± 8.90 | 402.39 ± 6.79 | 147.11 ± 7.12 |
| **TGFβ1** | 1,636.51 ± 16.26 | 1,166.88 ± 28.11 | 492.89 ± 7.70 | 270.24 ± 3.10 | 123.01 ± 1.75 |
| **TGFβ2** | 1,025.49 ±45.24 | 797.78 ± 19.23 | 338.44 ± 3.19 | 198.92 ± 11.96 | 125.88 ± 0.30 |
| **TGFβ3** | 1,067.12 ± 6.04 | 775.37 ± 11.07 | 427.90 ± 6.71 | 215.59 ± 7.26 | 169.46 ± 5.75 |
| **MMP-7** | 455.98 ± 28.24 | 321.48 ± 14.35 | 232.63 ± 11.15 | 142.07± 6.87 | 109.05 ± 6.68 |
| **MMP-9** | 733.62 ± 103.31 | 570.94 ± 79.44 | 267.29 ± 36.80 | 183.98 ± 28.86 | 156.83 ± 27.94 |
| **MMP-11** | 633.33 ± 48.27 | 520.05 ± 26.13 | 222.75 ± 7.85 | 169.57 ± 9.31 | 128.28 ± 9.06 |
| **MMP-14** | 865.63 ± 43.40 | 769.70 ± 49.74 | 323.25 ± 25.18 | 184.50 ± 15.26 | 153.36 ± 6.31 |
| **TNF-α** | 1,355.68 ± 183.07 | 980.46 ± 127.11 | 470.69 ± 62.10 | 218.38 ± 27.39 | 151.28 ± 22.25 |
| **VEGF** | 921,47 ± 72,63 | 575.62 ± 72.93 | 391.91 ± 49.18 | 200.18 ± 29.19 | 145.64 ± 16.94 |
| **Fibrosis** | 1,328.57 ± 265.80 | 1,127.78 ± 265.80 | 433.33 ± 115.47 | 233.33 ± 28.87 | 161.11 ± 34.69 |

| | | | | | |
|---|---|---|---|---|---|
| %Med P/C: percent increase in marker for the group treated with placebo with respect to the control group ± SD; %Med G5/C: percent increase in marker for the Group 5 with respect to the control group ± SD; %Med G6/C: percent increase in marker for the Group 6 with respect to the control group ± SD; %Med G7/C: percent increase in marker for the Group 7 with respect to the control group ± SD; %Med G8/C: percent increase in marker for the Group 8 with respect to the control group ± SD. | | | | | |

Similar results were observed in tissue samples obtained from the nasal cavity (*cf*. Tables 15 and 16), pharynx (*cf.* Tables 17 and 18), larynx (*cf.* Tables 19 and 20) and bronchi (*cf.* Tables 21 and 22).

**Table 15. Percent increase in marker for the placebo group and Groups 1 to 4 with respect to the control group ± SD in nasal cavity tissue.**

| | **%Med P/C** | **%Med G1/C** | **%Med G2/C** | **%Med G3/C** | **%Med G4/C** |
|---|---|---|---|---|---|
| **TGFα** | 3,509.80 ± 88.21 | 2,824.89 ± 194.95 | 1,907.98 ± 342.82 | 1,347.22 ± 145.56 | 952.16 ± 41.28 |
| **TGFβ1** | 2,773.08 ± 32.06 | 2,191.33 ± 144.90 | 1,562.26 ± 168.11 | 1,491.16 ± 66.00 | 1,373.96 ± 41.28 |
| **TGFβ2** | 1,940.80 ± 74.11 | 1,838.85 ± 69.75 | 1,469.70 ± 262.68 | 1,290.84 ± 88.45 | 1,151.85 ± 27.29 |
| **TGFβ3** | 3,187.67 ± 389.82 | 2,813.99 ± 342.04 | 2,128.24 ± 618.90 | 1,451.25 ± 88.45 | 1,188.89 ± 107.96 |
| **MMP-7** | 2,467.57 ± 478.29 | 2,267.10 ± 544.82 | 1,308.16 ± 288.79 | 1,119.62 ± 88.45 | 942.01 ± 291.79 |
| **MMP-9** | 1,531.63 ± 84.67 | 1,355.64 ± 71.12 | 768.75 ± 105.14 | 511.58 ± 112.67 | 328.92 ± 29,12 |
| **MMP-11** | 1,631.45 ± 37.29 | 1,023.73 ± 77.89 | 609.60 ± 138.77 | 392.70 ± 82.34 | 270.95 ± 28.58 |
| **MMP-14** | 1,422.66 ± 89.92 | 1,016.51 ± 67.99 | 680.45 ± 81.56 | 412.64 ± 82.34 | 317.68 ± 38.50 |
| **TNF-α** | 2,025.60 ± 75.38 | 1,411.41 ± 235.76 | 842.93 ± 81.56 | 658.65 ± 82.34 | 418.29 ± 68.03 |
| **VEGF** | 2,741.12 ± 219.07 | 1,293.11 ± 284.53 | 969.58 ± 81.56 | 716.62 ± 82.34 | 576,80 ± 40,97 |
| **Fibrosis** | 672.73 ± 128.11 | 513.89 ± 146.33 | 375.00 ± 81.56 | 300.00 ± 100.00 | 300.00 ± 100.00 |

| | | | | | |
|---|---|---|---|---|---|
| %Med P/C: percent increase in marker for the group treated with placebo with respect to the control group ± SD; %Med G1/C: percent increase in marker for the Group 1 with respect to the control group ± SD; %Med G2/C: percent increase in marker for the Group 2 with respect to the control group ± SD; %Med G3/C: percent increase in marker for the Group 3 with respect to the control group ± SD; %Med G4/C: percent increase in marker for the Group 4 with respect to the control group ± SD. | | | | | |

**Table 16. Percent increase in marker for the placebo group and Groups 5 to 8 with respect to the control group ± SD in nasal cavity tissue.**

| | **%Med P/C** | **%Med G5/C** | **%Med G6/C** | **%Med G7/C** | **%Med G8/C** |
|---|---|---|---|---|---|
| **TGFα** | 3,509.80 ± 88.21 | 2,908.21 ± 72.84 | 764.46 ± 39.44 | 495.68 ± 45.61 | 156.67 ± 12.98 |
| **TGFβ1** | 2,773.08 ± 32.06 | 2,065.52 ± 22.42 | 745.49 ± 11.90 | 480.80 ± 17.71 | 138.39 ± 9.96 |
| **TGFβ2** | 1,940.80 ± 74.11 | 1,451.21 ± 66.04 | 825.63 ± 34.06 | 252.52 ± 21.33 | 136.09 ± 7.57 |
| **TGFβ3** | 3,187.67 ± 389.82 | 2,168.67 ± 266.93 | 1,051.06 ± 34.06 | 482.03 ± 108.16 | 158.62 ± 15.53 |
| **MMP-7** | 2,467.57 ± 478.29 | 1,835.98 ± 342.42 | 1,147.87 ± 217.18 | 444.94 ± 102.68 | 136.90 ± 14.90 |
| **MMP-9** | 1,531.63 ± 84.67 | 1,289.77 ± 42.11 | 588.48 ± 34.52 | 291.36 ± 24.37 | 143.08 ± 12.14 |
| **MMP-11** | 1,631.45 ± 37.29 | 1,215.12 ± 32.39 | 489.76 ± 13.67 | 266.47 ± 19.16 | 133.33 ± 15.61 |
| **MMP-14** | 1,422.66 ± 89.92 | 1,190.13 ± 32.39 | 510.81 ± 36.50 | 206.80 ± 5.96 | 149.00 ± 14.70 |
| **TNF-α** | 2,025.60 ± 75.38 | 1,495.60 ± 32.39 | 736.83 ± 21.89 | 151.81 ± 7.84 | 122.23 ± 9.10 |
| **VEGF** | 2,741.12 ± 219.07 | 1,727.98 ± 142.31 | 1,010.56 ± 117.65 | 323.15 ± 15.27 | 145.68 ± 16.82 |
| **Fibrosis** | 672.73 ± 128.11 | 700.00 ± 90.14 | 319.44 ± 63.65 | 163.89 ±12.73 | 147.22 ± 20.97 |

| | | | | | |
|---|---|---|---|---|---|
| %Med P/C: percent increase in marker for the group treated with placebo with respect to the control group ± SD; %Med G5/C: percent increase in marker for the Group 5 with respect to the control group ± SD; %Med G6/C: percent increase in marker for the Group 6 with respect to the control group ± SD; %Med G7/C: percent increase in marker for the Group 7 with respect to the control group ± SD; %Med G8/C: percent increase in marker for the Group 8 with respect to the control group ± SD. | | | | | |

**Table 17. Percent increase in marker for the placebo group and Groups 1 to 4 with respect to the control group ± SD in pharyngeal tissue.**

| | **%Med P/C** | **%Med G1/C** | **%Med G2/C** | **%Med G3/C** | **%Med G4/C** |
|---|---|---|---|---|---|
| **TGFα** | 2,537.60 ± 87.23 | 2,274.76 ± 245.50 | 1,907.77 ± 434.67 | 1,780.45 ± 245.67 | 1,550.34 ± 34.23 |
| **TGFβ1** | 2,502.27 ± 45.36 | 2,198.45 ± 223.43 | 1,562.45 ± 212.34 | 1,208.45 ± 45.56 | 1,001.31 ± 36.23 |
| **TGFβ2** | 1,940.80 ± 74.11 | 1,575.67 ± 58.63 | 1,235.45 ± 262.68 | 980.65 ± 67.35 | 780.54 ± 34.81 |
| **TGFβ3** | 1,245.56 ± 202.78 | 967.72 ± 278.34 | 879.34 ± 109.34 | 659.59 ± 99.67 | 456.45 ± 78.45 |
| **MMP-7** | 2,324.56 ± 323.32 | 2,004.34 ± 381.48 | 1,591.34 ± 302.56 | 1,456.45 ± 156.51 | 967.12 ± 62.78 |
| **MMP-9** | 1,356.54 ± 76.54 | 1,102.28 ± 65.43 | 945.34 ± 125.34 | 789.45 ± 109.23 | 561.21 ± 89.23 |
| **MMP-11** | 1,789.56 ± 43.25 | 1,387.65 ± 89.45 | 1,980.45 ± 198.45 | 980.34 ± 78.37 | 670.45 ± 56.67 |
| **MMP-14** | 1,324.56 ± 67.23 | 1,148.43 ± 45.84 | 980.64 ± 67.65 | 762.45 ± 78.24 | 560.45 ± 56.34 |
| **TNF-α** | 2,123.55 ± 67.43 | 1,357.32 ± 234.43 | 987.63 ± 78.76 | 789.45 ± 45.35 | 418.29 ± 68.03 |
| **VEGF** | 2,546.45 ± 178.45 | 1,789.12 ± 299.45 | 1,256.67 ± 78.54 | 998.56 ± 67.24 | 567.45 ± 34.54 |
| **Fibrosis** | 605.23 ± 132.22 | 489.54 ± 122.44 | 457.45 ± 83.45 | 380.00 ± 78.00 | 275.00 ± 50.00 |

| | | | | | |
|---|---|---|---|---|---|
| %Med P/C: percent increase in marker for the group treated with placebo with respect to the control group ± SD; %Med G1/C: percent increase in marker for the Group 1 with respect to the control group ± SD; %Med G2/C: percent increase in marker for the Group 2 with respect to the control group ± SD; %Med G3/C: percent increase in marker for the Group 3 with respect to the control group ± SD; %Med G4/C: percent increase in marker for the Group 4 with respect to the control group ± SD. | | | | | |

**Table 18. Percent increase in marker for the placebo group and Groups 5 to 8 with respect to the control group ± SD in pharyngeal tissue.**

| | **%Med P/C** | **%Med G5/C** | **%Med G6/C** | **%Med G7/C** | **%Med G8/C** |
|---|---|---|---|---|---|
| **TGFα** | 2,537.60 ± 87.23 | 1,946.43± 89.40 | 1,364.46 ± 45.43 | 699.78 ± 56.53 | 101.34 ± 11.56 |
| **TGFβ1** | 2,502.27 ± 45.36 | 1,804.23 ± 45.34 | 1,220.34 ± 34.54 | 609.67 ± 45.34 | 121.89 ± 10.79 |
| **TGFβ2** | 1,940.80 ± 74.11 | 1,202.46 ± 45.34 | 725.63 ± 34.06 | 252.52 ± 21.33 | 136.09 ± 7.57 |
| **TGFβ3** | 1,245.56 ± 202.78 | 879.45 ± 99.56 | 543.45 ± 65.12 | 309.45 ± 33.25 | 112.43 ± 23.12 |
| **MMP-7** | 2,324.56 ± 323.32 | 1,820.78 ± 298.23 | 1,109.45 ± 112.32 | 450.45 ± 99.45 | 121.80 ± 10.34 |
| **MMP-9** | 1,356.54 ± 76.54 | 998.67 ± 43.23 | 505.32 ± 24.21 | 212.24 ± 17.21 | 121.23 ± 10.12 |
| **MMP-11** | 1,789.56 ± 43.25 | 1,324.23 ± 32.23 | 904.21 ± 21.47 | 529.34 ± 15.45 | 109.43 ± 9.43 |
| **MMP-14** | 1,324.56 ± 67.23 | 1,091.23 ± 51.12 | 709.21 ± 32.50 | 245.45 ± 21.20 | 135.00 ± 12.42 |
| **TNF-α** | 2,123.55 ± 67.43 | 1,532.34 ± 56.56 | 989.36 ± 34.32 | 298.67 ± 23.21 | 167.34 ± 12.21 |
| **VEGF** | 2,546.45 ± 178.45 | 1,858.37 ± 112.25 | 1,198.56 ± 94.32 | 654.32 ± 11.32 | 121.34 ± 9.45 |
| **Fibrosis** | 605.23 ± 132.22 | 480.30 ± 90.50 | 258.34 ± 32.35 | 152.23 ± 11.54 | 128.32 ± 9.87 |

| | | | | | |
|---|---|---|---|---|---|
| %Med P/C: percent increase in marker for the group treated with placebo with respect to the control group ± SD; %Med G5/C: percent increase in marker for the Group 5 with respect to the control group ± SD; %Med G6/C: percent increase in marker for the Group 6 with respect to the control group ± SD; %Med G7/C: percent increase in marker for the Group 7 with respect to the control group ± SD; %Med G8/C: percent increase in marker for the Group 8 with respect to the control group ± SD. | | | | | |

**Table 19. Percent increase in marker for the placebo group and Groups 1 to 4 with respect to the control group ± SD in laryngeal tissue.**

| | **%Med P/C** | **%Med G1/C** | **%Med G2/C** | **%Med G3/C** | **%Med G4/C** |
|---|---|---|---|---|---|
| **TGFα** | 2,450.34 ± 56.12 | 1,875.56 ± 321.49 | 1,500.45 ± 248.54 | 1,320.32 ± 145.59 | 1,101.28 ± 87.43 |
| **TGFβ1** | 1,958.34 ± 54.32 | 1,567.67 ± 289.56 | 1,278.89 ± 199.67 | 1,067.78 ± 156.89 | 988.78 ± 99.87 |
| **TGFβ2** | 2,105.45 ± 88.66 | 1,789.56 ± 390.89 | 1,398.89 ± 270.78 | 989.89 ± 178.17 | 816.89 ± 120.45 |
| **TGFβ3** | 1,545.67 ± 126.54 | 1,234.56 ± 399.89 | 1,009.78 ± 278.90 | 899.98 ± 125.67 | 567.89 ± 99.89 |
| **MMP-7** | 1,567.45 ± 234.34 | 1,012.67 ± 389.78 | 899.78 ± 280.78 | 788.56 ± 198.78 | 576.89 ± 101.89 |
| **MMP-9** | 1,367.67 ± 78.34 | 1,290.89 ± 432.12 | 983.98 ± 348.78 | 898.18 ± 198.89 | 788.78 ± 99.10 |
| **MMP-11** | 987.45 ± 35.27 | 789.61 ± 265.28 | 576.17 ± 151.89 | 456.89 ± 123.89 | 324.89 ± 98.45 |
| **MMP-14** | 878.35 ± 45.32 | 678.45 ± 134.83 | 567.34 ± 110.56 | 459.43 ± 89.11 | 387.34 ± 63.80 |
| **TNF-α** | 1,234.55 ± 54.32 | 1,078.45 ± 289.45 | 809.99 ± 178.39 | 708.73 ± 123.75 | 509.87 ± 98.80 |
| **VEGF** | 1,467.56 ± 98.34 | 1,134.34 ± 239.45 | 980.54 ± 128.49 | 875.80 ± 112.76 | 667.82 ± 93.12 |
| **Fibrosis** | 500.50 ± 100.00 | 250.50 ± 100.30 | 225.70 ± 50.50 | 200.57 ± 50.10 | 150.70 ± 40.50 |

| | | | | | |
|---|---|---|---|---|---|
| %Med P/C: percent increase in marker for the group treated with placebo with respect to the control group ± SD; %Med G1/C: percent increase in marker for the Group 1 with respect to the control group ± SD; %Med G2/C: percent increase in marker for the Group 2 with respect to the control group ± SD; %Med G3/C: percent increase in marker for the Group 3 with respect to the control group ± SD; %Med G4/C: percent increase in marker for the Group 4 with respect to the control group ± SD. | | | | | |

**Table 20. Percent increase in marker for the placebo group and Groups 5 to 8 with respect to the control group ± SD in laryngeal tissue.**

| | **%Med P/C** | **%Med G5/C** | **%Med G6/C** | **%Med G7/C** | **%Med G8/C** |
|---|---|---|---|---|---|
| **TGFα** | 2,450.34 ± 56.12 | 1,820.45 ± 102.34 | 1,250,74 ± 45,80 | 783,78 ± 25,74 | 234.67 ± 12.23 |
| **TGFβ1** | 1,958.34 ± 54.32 | 1,398.78 ± 98.78 | 760.87 ± 52.76 | 320.34 ± 23.63 | 102.23 ± 10.60 |
| **TGFβ2** | 2,105.45 ± 88.66 | 1,506.89 ± 101.45 | 996.89 ± 56.49 | 560.38 ± 24.12 | 120.23 ± 10.90 |
| **TGFβ3** | 1,545.67 ± 126.54 | 1,183.45 ± 67.39 | 702.33 ± 34.12 | 305.32 ± 15.12 | 132.22 ± 12.56 |
| **MMP-7** | 1,567.45 ± 234.34 | 1,205.45 ± 34.48 | 809.54 ± 23.84 | 372.23 ± 14.23 | 112.25 ± 9.25 |
| **MMP-9** | 1,367.67 ± 78.34 | 989.34 ± 43.12 | 567.37 ± 23.54 | 256.47 ± 12.45 | 123.23 ± 24.54 |
| **MMP-11** | 987.45 ± 35.27 | 768.34 ± 23.67 | 504.34 ± 12.32 | 203.23 ± 10.56 | 167.36 ± 9.50 |
| **MMP-14** | 878.35 ± 45.32 | 639.34 ± 32.23 | 543.12 ± 21.23 | 342.23 ± 12.34 | 201.28 ± 8.32 |
| **TNF-α** | 1,234.55 ± 54.32 | 879.34 ± 34.12 | 456.34 ± 26.27 | 323.12 ± 21.23 | 150.34 ± 14.23 |
| **VEGF** | 1,467.56 ± 98.34 | 950.34 ± 32.21 | 670.32 ± 21.25 | 570.12 ± 14.34 | 250.21 ± 10.24 |
| **Fibrosis** | 500.50 ± 100.00 | 375.50 ± 100.00 | 275.45 ± 50.50 | 200.60 ± 20.20 | 125.40± 10.10 |

| | | | | | |
|---|---|---|---|---|---|
| %Med P/C: percent increase in marker for the group treated with placebo with respect to the control group ± SD; %Med G5/C: percent increase in marker for the Group 5 with respect to the control group ± SD; %Med G6/C: percent increase in marker for the Group 6 with respect to the control group ± SD; %Med G7/C: percent increase in marker for the Group 7 with respect to the control group ± SD; %Med G8/C: percent increase in marker for the Group 8 with respect to the control group ± SD. | | | | | |

**Table 21. Percent increase in marker for the placebo group and Groups 1 to 4 with respect to the control group ± SD in bronchial tissue.**

| | **%Med P/C** | **%Med G1/C** | **%Med G2/C** | **%Med G3/C** | **%Med G4/C** |
|---|---|---|---|---|---|
| **TGFα** | 2,345.45 ± 34.12 | 1,980.45 ± 345.23 | 1,540.45 ± 250.45 | 1,190.45 ± 178.12 | 873.34 ± 123.34 |
| **TGFβ1** | 1,569.34 ± 45.67 | 1,234.56 ± 159.34 | 984.45 ± 98.67 | 768.32 ± 76.35 | 502.23 ± 34.21 |
| **TGFβ2** | 1,789.56 ± 34.12 | 1,342.12 ± 112.45 | 1,008.32 ± 88.43 | 878.34 ± 78.23 | 457.45 ± 45.67 |
| **TGFβ3** | 897.45 ± 89.45 | 672.56 ± 98.45 | 452.45 ± 56.32 | 345.32 ± 45.32 | 236.12 ± 39.54 |
| **MMP-7** | 762.34 ± 34.56 | 504.23 ± 83.46 | 406.34 ± 58.45 | 398.45 ± 45.65 | 289.56 ± 32.21 |
| **MMP-9** | 984.34 ± 57.34 | 749.34 ± 145.43 | 672.94 ± 93.92 | 495.87 ± 65.45 | 305.34 ± 45.82 |
| **MMP-11** | 894.56 ± 78.45 | 784.45 ± 121.63 | 673.45 ± 85.46 | 563.45 ± 67.43 | 453.34 ± 78.54 |
| **MMP-14** | 678.45 ± 45.39 | 567.32 ± 132.45 | 434.45 ± 89.45 | 325.56 ± 43.24 | 312.56 ± 56.43 |
| **TNF-α** | 898.34 ± 34.23 | 789.45 ± 201.36 | 580.67 ± 145.67 | 467.45 ± 112.56 | 356.34 ± 89.45 |
| **VEGF** | 567.23 ± 28.67 | 457.34 ± 145.34 | 356.45 ± 95.56 | 256.34 ± 67.89 | 203.56 ± 35.56 |
| **Fibrosis** | 3,000.00 ± 00.00 | 2,500.10 ± 100.50 | 2,000.00 ± 00.00 | 1,500.00 ± 00.00 | 1,000.00 ± 00.00 |

| | | | | | |
|---|---|---|---|---|---|
| %Med P/C: percent increase in marker for the group treated with placebo with respect to the control group ± SD; %Med G1/C: percent increase in marker for the Group 1 with respect to the control group ± SD; %Med G2/C: percent increase in marker for the Group 2 with respect to the control group ± SD; %Med G3/C: percent increase in marker for the Group 3 with respect to the control group ± SD; %Med G4/C: percent increase in marker for the Group 4 with respect to the control group ± SD. | | | | | |

**Table 22. Percent increase in marker for the placebo group and Groups 5 to 8 with respect to the control group ± SD in bronchial tissue.**

| | **%Med P/C** | **%Med G5/C** | **%Med G6/C** | **%Med G7/C** | **%Med G8/C** |
|---|---|---|---|---|---|
| **TGFα** | 2,345.45 ± 34.12 | 1,875.75 ± 60.30 | 1,267.45 ± 38.32 | 689.78 ± 38.72 | 209.45 ± 12.45 |
| **TGFβ1** | 1,569.34 ± 45.67 | 1,103.34 ± 23.43 | 708.45 ± 16.45 | 302.34 ± 12.47 | 124.37 ± 9.7 |
| **TGFβ2** | 1,789.56 ± 34.12 | 1,209.34 ± 76.45 | 878.45 ± 32.21 | 328.45 ± 15.34 | 126.45 ± 10.23 |
| **TGFβ3** | 897.45 ± 89.45 | 604.34 ± 102.34 | 439.53 ± 50.47 | 234.56 ± 43.89 | 179.19 ± 23.21 |
| **MMP-7** | 762.34 ± 34.56 | 549.34 ± 75.45 | 467.45 ± 56.45 | 356.47 ± 43.21 | 241.56 ± 23.24 |
| **MMP-9** | 984.34 ± 57.34 | 673.45 ± 34.12 | 452.61 ± 27.73 | 325.54 ± 20.21 | 153.34 ± 45.36 |
| **MMP-11** | 894.56 ± 78.45 | 653.23 ± 67.38 | 438.43 ± 51.23 | 342.56 ± 34.23 | 159.42 ± 34.25 |
| **MMP-14** | 678.45 ± 45.39 | 458.45 ± 34.31 | 342.32 ± 24.32 | 246.47 ± 17.28 | 164.63 ± 12.53 |
| **TNF-α** | 898.34 ± 34.23 | 673.34 ± 51.36 | 451.23 ± 45.32 | 325.21 ± 23.21 | 201.23 ± 14.24 |
| **VEGF** | 567.23 ± 28.67 | 324.53 ± 43.32 | 235.71 ± 24.17 | 153.57 ± 16.42 | 112.71 ± 8.23 |
| **Fibrosis** | 3,000.00 ± 00.00 | 2,000.00 ± 00.00 | 1,000.00 ± 00.00 | 500.00 ± 00.00 | 100.50 ± 50.50 |

| | | | | | |
|---|---|---|---|---|---|
| %Med P/C: percent increase in marker for the group treated with placebo with respect to the control group ± SD; %Med G5/C: percent increase in marker for the Group 5 with respect to the control group ± SD; %Med G6/C: percent increase in marker for the Group 6 with respect to the control group ± SD; %Med G7/C: percent increase in marker for the Group 7 with respect to the control group ± SD; %Med G8/C: percent increase in marker for the Group 8 with respect to the control group ± SD. | | | | | |

## Claims

1. A pharmaceutical composition suitable for inhalation, wherein
the pharmaceutical composition comprises a neurokinin-1 (NK1) receptor antagonist and at least one excipient, and
the neurokinin-1 (NK1) receptor antagonist is aprepitant and the at least one excipient is olive oil.

2. The pharmaceutical composition according to claim 1, which is suitable for administration as an inhaler spray, aerosol or nebulized administration form, using an inhaler or nebulizer as an administration device.

3. The pharmaceutical composition according to claim 1 or 2, which further comprises ethanol.

4. A pharmaceutical composition according to any one of claims 1 to 3, for use in the treatment of at least one disease of the respiratory tract.

5. The pharmaceutical composition for use according to claim 4, wherein said at least one disease of the respiratory tract is selected from cancer, an inflammatory condition and an inflammatory-fibrosis condition.

6. The pharmaceutical composition for use according to claim 5, wherein said at least one disease of the respiratory tract is a cancer selected from lung cancer, tracheal cancer, laryngeal cancer, pharyngeal cancer, cancer of the nasal cavities and esophageal cancer.

7. The pharmaceutical composition for use according to claim 5, wherein said at least one disease of the respiratory tract is an inflammatory condition selected from rhinitis, glossitis, buccal aphthous stomatitis, gingivitis, pharyngitis, laryngitis, bronchitis and alveolitis.

8. The pharmaceutical composition for use according to claim 5, wherein said at least one disease of the respiratory tract is an inflammatory-fibrosis condition selected from usual interstitial pneumonia, idiopathic interstitial pneumonias and pneumoconiosis.

9. The pharmaceutical composition for use according to any one of claims 4 to 8, which is administered by inhalation.

10. Kit of parts for administering a pharmaceutical composition, comprising:
i) a container comprising the pharmaceutical composition according to any of claims 1 to 3; and
ii) an administration device for administering said composition as an inhalant, nebulization, vapor, smoke, aerosol, pulverization or spray from said container.

11. Kit of parts according to claim 10, wherein the administration device is an inhaler, nebulizer, vaporizer, electronic cigarette, aerosol canister or bottle, atomizer or ventilator.

12. A method for producing the pharmaceutical composition according to any of claims 1 to 3, which comprises mixing:
a) a neurokinin-1 (NK1) receptor antagonist which is aprepitant; and
b) at least one excipient which is olive oil.

## Patentansprüche

1. Inhalationsfähige pharmazeutische Zusammensetzung, wobei
die pharmazeutische Zusammensetzung einen Neurokinin-1-(NK1) Rezeptorantagonisten und mindestens einen Hilfsstoff umfasst und
der Neurokinin-1- (NK1) Rezeptorantagonist Aprepitant ist und der mindestens eine Hilfsstoff Olivenöl ist.

2. Pharmazeutische Zusammensetzung nach Anspruch 1, die zur Verabreichung als Inhalationsspray, Aerosol oder vernebelte Verabreichungsform unter Verwendung eines Inhalators oder Zerstäubers als Verabreichungsvorrichtung geeignet ist.

3. Pharmazeutische Zusammensetzung nach Anspruch 1 oder 2, die ferner Ethanol umfasst.

4. Pharmazeutische Zusammensetzung nach einem der Ansprüche 1 bis 3 zur Verwendung bei der Behandlung von mindestens einer Erkrankung der Atemwege.

5. Pharmazeutische Zusammensetzung zur Verwendung nach Anspruch 4, wobei die mindestens eine Erkrankung der Atemwege aus Krebs, einer entzündlichen Erkrankung und einer entzündlichen Fibroseerkrankung ausgewählt ist.

6. Pharmazeutische Zusammensetzung zur Verwendung nach Anspruch 5, wobei die mindestens eine Erkrankung der Atemwege ein Krebs ist, der aus Lungenkrebs, Trachealkrebs, Kehlkopfkrebs, Pharyngealkrebs, Krebs der Nasenhöhlen und Speiseröhrenkrebs ausgewählt ist.

7. Pharmazeutische Zusammensetzung zur Verwendung nach Anspruch 5, wobei die mindestens eine Erkrankung der Atemwege eine entzündliche Erkrankung ist, die aus Rhinitis, Glossitis, bukkaler aphthöser Stomatitis, Gingivitis, Pharyngitis, Laryngitis, Bronchitis und Alveolitis ausgewählt ist.

8. Pharmazeutische Zusammensetzung zur Verwendung nach Anspruch 5, wobei die mindestens eine Erkrankung der Atemwege eine entzündliche Fibrose ist, die aus üblicher interstitieller Pneumonie, idiopathischer interstitieller Pneumonie und Pneumokoniose ausgewählt ist.

9. Pharmazeutische Zusammensetzung zur Verwendung nach einem der Ansprüche 4 bis 8, die durch Inhalation verabreicht wird.

10. Teilekit zur Verabreichung einer pharmazeutischen Zusammensetzung, das Folgendes umfasst:
i) einen Behälter, der die pharmazeutische Zusammensetzung nach einem der Ansprüche 1 bis 3 umfasst; und
ii) eine Verabreichungsvorrichtung zum Verabreichen der Zusammensetzung als Inhalationsmittel, Zerstäubung, Dampf, Rauch, Aerosol, Pulverisierung oder Spray aus dem Behälter.

11. Teilekit nach Anspruch 10, wobei die Verabreichungsvorrichtung ein Inhalator, ein Zerstäuber, ein Verdampfer, eine elektronische Zigarette, ein Aerosolbehälter oder eine Flasche, ein Zerstäuber oder ein Beatmungsgerät ist.

12. Verfahren zur Herstellung der pharmazeutischen Zusammensetzung nach einem der Ansprüche 1 bis 3, das das Mischen von Folgendem umfasst:
a) eines Neurokinin-1- (NK1) Rezeptorantagonisten, der Aprepitant ist; und
b) mindestens eines Hilfsstoffs, der Olivenöl ist.

## Revendications

1. Composition pharmaceutique appropriée pour l'inhalation, dans laquelle
la composition pharmaceutique comprend un antagoniste du récepteur de la neurokinine-1 (NK1) et au moins un excipient, et
l'antagoniste du récepteur de la neurokinine-1 (NK1) est l'aprépitant et l'au moins un excipient est l'huile d'olive.

2. Composition pharmaceutique selon la revendication 1, qui convient pour une administration sous forme vaporisation d'inhalateur, d'aérosol ou de nébulisation, en utilisant un inhalateur ou un nébuliseur comme dispositif d'administration.

3. Composition pharmaceutique selon la revendication 1 ou 2, qui comprend en outre de l'éthanol.

4. Composition pharmaceutique selon l'une quelconque des revendications 1 à 3, destinée à utiliser dans le traitement d'au moins une maladie des voies respiratoires.

5. Composition pharmaceutique à utiliser selon la revendication 4, dans laquelle ladite au moins une maladie des voies respiratoires est choisie parmi le cancer, un état inflammatoire et un état de fibrose inflammatoire.

6. Composition pharmaceutique à utiliser selon la revendication 5, dans laquelle ladite au moins une maladie des voies respiratoires est un cancer choisi parmi le cancer du poumon, le cancer de la trachée, le cancer du larynx, le cancer du pharynx, le cancer des fosses nasales et le cancer de l'œsophage.

7. Composition pharmaceutique à utiliser selon la revendication 5, dans laquelle ladite au moins une maladie des voies respiratoires est une affection inflammatoire choisie parmi la rhinite, la glossite, la stomatite aphteuse buccale, la gingivite, la pharyngite, la laryngite, la bronchite et l'alvéolite.

8. Composition pharmaceutique à utiliser selon la revendication 5, dans laquelle ladite au moins une maladie des voies respiratoires est une affection de fibrose inflammatoire choisie parmi la pneumonie interstitielle usuelle, les pneumonies interstitielles idiopathiques et la pneumoconiose.

9. Composition pharmaceutique à utiliser selon l'une quelconque des revendications 4 à 8, qui est administrée par inhalation.

10. Kit de composantes pour l'administration d'une composition pharmaceutique, comprenant :
i) un récipient comprenant la composition pharmaceutique selon l'une quelconque des revendications 1 à 3 ; et
ii) un dispositif d'administration pour administrer ladite composition sous forme d'inhalant, de nébulisation, de vapeur, de fumée, d'aérosol, de pulvérisation ou de vaporisation à partir dudit récipient.

11. Kit de composantes selon la revendication 10, dans lequel le dispositif d'administration est un inhalateur, un nébuliseur, un vaporisateur, une cigarette électronique, une cartouche ou un flacon aérosol, un atomiseur ou un ventilateur.

12. Procédé de production de la composition pharmaceutique selon l'une quelconque des revendications 1 à 3, qui comprend le mélange :
a) d'un antagoniste du récepteur de la neurokinine-1 (NK1) qui est l'aprépitant ; et
b) au moins un excipient qui est l'huile d'olive.
